# EUROPÄISCHE PATENTANMELDUNG

(11) **EP 4 635 491 A1**
(43) Veröffentlichungstag der Anmeldung: **22.10.2025**
(21) Anmeldenummer: 25168077.3
(22) Anmeldetag: 02.04.2025
(51) Int. Cl.: A61K 31/21, A61J 1/00, A61K 33/00, A61K 33/14, A61K 33/42, A61K 36/185, A61P 21/00

(54) **ZUSAMMENSETZUNG, DIE EINEN TRÄGER UND ENTWEDER NATRIUMCHLORID ODER KALIUMCHLORID ENTHÄLT, SOWIE APPLIKATOR MIT EINER DRUCK-AUFTRAGUNGSEINHEIT ZUM AUFTRAGEN DER ZUSAMMENSETZUNG SOWIE SET AUS EINEM ERSTEN UND ZWEITEN APPLIKATOR**

(30) Priorität: 03.04.2024 DE 102024109321
(71) Anmelder: Gemeinschaftspraxis für Physiotherapie GbR (vertretungsberechtigte Gesellschafter: Natalie Morell und Nils Morell), 31785 Hameln (DE)
(72) Erfinder: Morell, Stephanie, verstorben (DE); Morell, Natalie, 31785 Hameln (DE); Morell, Nils-Oliver, 31785 Hameln (DE)
(74) Vertreter: Gerstein, Hans Joachim

(57) **Zusammenfassung**

Die vorliegende Erfindung offenbart eine Zusammensetzung, die einen Träger und entweder Natriumchlorid oder Kaliumchlorid enthält, zur Verwendung für die Auflösung von Faszienverkürzungen, indem die Zusammensetzung im Bereich des Nervus trigeminus, des Nervus facialis, des Nervus medianus, des Nervus ischiadicus oder auf eine Kombination aus mindestens zwei dieser Bereiche auf die Haut aufgetragen wird.

## Beschreibung

Die vorliegende Erfindung betrifft eine Zusammensetzung zum Auftragen auf die Haut zur Auflösung von Faszienverkürzungen, die einen Träger und entweder Natriumchlorid oder Kaliumchlorid enthält. Darüber hinaus wird eine besondere Verwendung der Zusammensetzung mit einem Applikator bereitgestellt, der es ermöglicht die erfindungsgemäße Zusammensetzung zur Auflösung von Faszienverkürzungen auf die Haut aufzutragen. Schließlich wird eine besondere Verwendung der Zusammensetzung mit einem Set aus einem ersten, erfindungsgemäßen Applikator, der die Natriumchlorid enthaltende Zusammensetzung beinhaltet und einem zweiten, erfindungsgemäßen Applikator, der die Kaliumchlorid enthaltende Zusammensetzung beinhaltet, beansprucht.

### Stand der Technik

Die Physiotherapie beschäftigt sich seit langem mit der Erkennung und Behandlung von sogenannten Triggerpunkten. Dabei handelt es sich um knotenartige, tastbare Verdickungen im Gewebe von Skelettmuskeln, die zu großflächig ausstrahlenden Schmerzen, zu starkem Kraftverlust und damit verbundenen Einschränkungen der Bewegung führen können. Interessanterweise reicht bereits eine geringe manuelle Druckausübung auf den Triggerpunkt aus, um beim Betroffenen einen starken Schmerzreiz auszulösen. Der Fachmann unterscheidet dabei zwischen diesen latenten Triggerpunkten, die nur auf Berührung und Druck reagieren, und aktiven Triggerpunkten, die sowohl in Ruhe, als auch in Bewegung, starke Schmerzen verursachen können. Triggerpunkte können dabei in allen möglichen Muskeln des menschlichen Körpers auftreten.

Während die Existenz der Triggerpunkte klinisch bewiesen ist, gibt es Unklarheiten bezüglich ihrer Entstehung.

Travell, Simons & Simons: "Myofascial Pain and Dysfunction", 3rd Edition, Wolters Kluver 2019 beschreibt Triggerpunkte und ihren Zusammenhang mit motorischen Endplatten. Aus mechanischer Sicht wird eine Behandlung mit trockenen Nadeln zur Störung der Integrität der dysfunktionalen Endplatten, Zunahme der Sarkomerlänge und Verringerung der Überlappung zwischen Aktin- und Myosinfilamenten vorgeschlagen. Auch eine Inaktivierung von Triggerpunkten bspw. mit Botox ist denkbar, da dieses eine spezifische pharmakologische Wirkung auf die motorische Endplatte hat und eine wirksame Lösung für Patienten mit myofaszialen Schmerzen sein kann. Es wird davon ausgegangen, dass eine fehlgesteuerte motorische Endplatte maßgeblich an der Formation von Triggerpunkten beteiligt ist.

Durch monotone Bewegungsabläufe im Alltag (z. B. Arbeiten am PC mit Maus und Tastatur) oder eine ungesunde Körperhaltung kann es zu Fehl- oder Überbelastung der Muskulatur kommen. Die daraus resultierenden Läsionen im Muskelgewebe schädigen die motorische Endplatte, die dadurch erhöhte Mengen des Neurotransmitters Acetylcholin ausschüttet. Durch diese pathologisch gesteigerte Ausschüttung kommt es zur Dauer-Depolarisation der Membran, die wiederum zu einer Dauerkontraktion des betroffenen Muskelbereichs führt. In Folge werden Blutkapillare dauerhaft komprimiert. Die Kontraktion des Muskels erhöht den Energie- und Sauerstoffbedarf im beteiligten Gewebe. Gleichzeitig ist die Versorgung durch die komprimierten Blutkapillare nicht mehr gegeben und der Zustand verstärkt sich von selbst. Die resultierende Hypoxie führt zur Freisetzung von Botenstoffen im betroffenen Bereich, die die Schmerzrezeptoren sensibilisieren.

Neben diesen muskulären, motorischen Beschwerden können Triggerpunkte, je nach Entstehungsort, auch neurologische, sensorische Beschwerden auslösen. So werden von Betroffenen unter anderem Veränderungen des Sichtfelds, erhöhter Speichelfluss, sowie Veränderungen des Gleichgewichtsgefühls, des Hörvermögens und Schwindel beschrieben.

Eine gängige Methode um Triggerpunkte zu therapieren ist die Triggerpunktmassage. Der genaue Wirkmechanismus ist dabei nicht abschließend geklärt, jedoch ist der Nutzen empirisch belegt. Durch kontrollierte und andauernde Druckausübung kann der Triggerpunkt aufgelöst werden. Die Massage kann dabei manuell oder mit Hilfe eines Hilfsmittels vom Fachmann durchgeführt werden. Eine Eigenanwendung der Hilfsmittel ist ebenfalls möglich.

Die US 9 693 929 B2 beschreibt ein Massagegerät zur Eigenbehandlung von Triggerpunkten. Die Vorrichtung besteht aus einer Spindel und einer drehbaren Rolle, die mit einem Mantel versehen ist. Dieser weist eine strukturierte Oberfläche auf, die eine massierende Wirkung bei Kontakt mit der Haut zeigt. Zusätzlich lässt sich der Mantel erwärmen oder abkühlen, um so die Durchblutung der Haut anzuregen.

In der US 2018/0028396 A1 wird eine Vorrichtung zur Eigenbehandlung von Triggerpunkten beschrieben, die aus einer Hülle und zwei Bällen besteht. Die Vorrichtung wird zwischen einer harten Oberfläche und dem Anwender platziert. Durch Bewegungen des Anwenders entfalten die Bälle eine massierende Wirkung im behandelten Körperteil. Um diesen Effekt noch weiter zu verstärken, lassen sich die Bälle durch eine eingebaute Heizung erwärmen und können zur Vibration gebracht werden.

In einem anderen Verfahren werden Triggerpunkte mit Hilfe von Nadeln, ähnlich einer Akkupunktur, behandelt. Beim sogenannten "Dry Needling" werden wiederholt feine Nadelstiche in den Triggerpunkt gesetzt. Durch den mechanischen Reiz soll sich die knotenartige Verhärtung im Muskel auflösen. Diese Methode kann noch mit der gleichzeitigen Injektion von lokalwirkenden Anästhetika kombiniert werden, um die von den Triggerpunkten verursachten Schmerzen vorübergehend zu betäuben.

Die beschriebenen Methoden zielen darauf ab, den Triggerpunkt auf muskulärer Ebene aufzulösen. Dies bringt gewisse Nachteile mit sich. So wird die Triggerpunktmassage von Patienten als extrem schmerzhaft beschrieben. Wenn man bedenkt, dass bereits leichter Druck zu einem ausstrahlenden Schmerzreiz führt, so erscheint dies sehr nachvollziehbar. Dies senkt die Akzeptanz des Patienten und kann den Erfolg der Anwendung schmälern, vor allem wenn eine Eigenanwendung angestrebt ist. Die Nadel-Behandlung von Triggerpunkten wird als weniger unangenehm beschrieben. Jedoch liegt durch die Injektionen ein erhöhtes Infektionsrisiko vor. Aufgrund dessen scheidet die Eigenanwendung des Verfahrens aus. Darüber hinaus ist die Verwendung von Lokalanästhetika nur eine vorübergehende Lösung zur Linderung von akuten Beschwerden.

US 2020/0383660 A1 beschreibt einen Ultraschall-Applikator mit einem Reservoir, das ein Ultraschall-Gel aufnimmt.

US 2021/0228718 A1 offenbart die Verwendung von Wasser als ein Medikament, wobei das Wasser mit elektromagnetischer Energie beaufschlagt wird. Hierzu wird Lichtenergie oder elektrische Energie gepulst mit zwei Frequenzen in das Wasser eingebracht.

WO 2020/163143 A1 offenbart topische Formulierungen zur Schmerzlinderung und Schlafhilfe, die Kaliumsalze als Wirkstoff verwenden. Die Formulierung enthält als Wirkstoff ein Kaliumion , das durch ein Anion elektrisch ausgeglichen wird, wobei das Anion aus der GRAS-Liste der FDA ausgewählt ist. Das (Gegen-)Anion kann ein Phosphat, Citrat, Acetat, Chlorid, Carbonat oder Bicarbonat sein. Ein Ungleichgewicht zwischen dem Säure-Base-Charakter des K⁺ und des Gegenanions kann durch Einstellen des pH-Werts der Zusammensetzung auf einen nahezu neutralen pH-Wert ausgeglichen werden. Der Zusammensetzung kann ein Tensid zugesetzt werden, das die Permeation von K⁺ in die Haut verbessern kann.

Die ursprünglichen Ursachen der Entstehung von Triggerpunkten werden bisher außer Acht gelassen. Diese Ursachen können entweder neurologischen oder faszialen Ursprungs sein. Durch den Ansatz Triggerpunkte auf muskulärer Ebene auflösen zu wollen, kann es schnell zu einem Rückfall kommen, da die eigentliche Ursache nicht behoben ist, sondern nur die Symptome behandelt wurden.

### Beschreibung der Erfindung

Aufgabe der Erfindung ist die Bereitstellung einer Zusammensetzung zur Auftragung auf die Haut zur Auflösung von Faszienverkürzungen und ein hierzu ausgebildeter Applikator, die die oben genannten Nachteile des Standes der Technik vermindern und verringern soll.

Die Aufgabe wird durch die Zusammensetzung mit den Merkmalen des Anspruchs 1, die Verwendung einer solchen Zusammensetzung für die Auflösung von Faszienverkürzungen durch Auftragen mit einem Applikator mit den Merkmalen des Anspruchs 8 und 9 sowie durch die Verwendung einer solchen Zusammensetzung für die Auflösung von Faszienverkürzungen durch Auftragen mit dem Set mit den Merkmalen des Anspruchs 11 gelöst. Vorteilhafte Ausführungsformen sind in den Unteransprüchen beschrieben.

Die vorliegende Erfindung löst das Problem durch eine Zusammensetzung, die einen Träger und entweder Natriumchlorid oder Kaliumchlorid enthält. Die Zusammensetzung wird zur Auflösung von Faszienverkürzungen verwendet, indem die Zusammensetzung im Bereich des Nervus trigeminus, des Nervus facialis, des Nervus medianus, des Nervus ischiadicus oder auf eine Kombination aus mindestens zwei dieser Bereiche auf die Haut aufgetragen wird.

Als Faszienverkürzung ist dabei die Faszienanhaftung unter Beteiligung eines Zweigs des Nervus trigeminus, des Nervus facialis, des Nervus medianus oder des Nervus ischiadicus an eine jeweils angrenzende Knochenhaut zu verstehen. Dabei entsteht ein Tenderpunkt, also eine knotenartige Verdickung im Bereich des Muskel-Sehnenübergangs, der schmerzhaft auf manuelle Druckausübung reagiert. Da eine direkte Verbindung zwischen der verkürzten Faszie und dem zugehörigen Muskel besteht, kommt es gleichzeitig zu einer Muskelverkürzung. Faszienverkürzung und Muskelverkürzung ziehen eine Körperasymmetrie nach sich, die ungleichmäßige Bewegungsabläufe verursacht. Diese Körperasymmetrie ist gleichzusetzen mit einer einseitigen Skoliose und gleichzeitiger Beinlängendifferenz. Dies bewirkt die Formation von Triggerpunkten in betroffenen Muskel an unterschiedlichen Stellen im Körper, die dann je nach Entstehungsort, motorische oder neurologische Beschwerden hervorrufen. Das heißt, dass es sich bei der Faszienverkürzung durch eine Faszienanhaftung an eine angrenzende Knochenhaut unter der Beteiligung mindestens eines Zweigs des Nervus trigeminus, des Nervus facialis, des Nervus medianus oder des Nervus ischiadicus, insbesondere unter Entstehung eines Tenderpunkts, um den faszialen Ursprung der Triggerpunkt-Formation handelt. Es ist möglich, dass die Lokalisierung der Faszienverkürzung dabei identisch mit dem Auftreten mindestens eines Tenderpunkts im betroffenen Muskel ist. Es ist wiederum auch möglich, dass eine Faszienverkürzung vorliegt, die nicht zur Ausbildung eines Tenderpunkts im betroffenen Muskel führt.

Die aus der Faszienverkürzung resultierende Nervenbahnblockade mindestens eines Zweigs des Nervus trigeminus, des Nervus facialis, des Nervus medianus oder des Nervus ischiadicus ist dabei als die neurologische Komponente der Triggerpunkt-Formation zu verstehen.

Damit ist die Verwendung zur Auflösung von Faszienverkürzungen im Sinne der Verwendung zur primären Auflösung von Faszienverkürzungen, Muskelverkürzungen und/oder Nervenbahnblockaden zu verstehen, wobei damit immer eine sekundäre Verwendung mindestens zur Auflösung von Faszienverkürzungen als ursächliche Funktionsstörung verbunden ist.

Als Nervenbahnblockade ist dabei der gestörte Zustand eines Nervs zu verstehen, der letztlich durch eine chronisch aktivierte motorische Endplatte an der Ausbildung eines Triggerpunktes im Muskel beteiligt ist. Es ist möglich, dass die Lokalisierung der Nervenbahnblockade des gestörten Nervs identisch mit der Lokalisierung der chronisch aktivierten motorischen Endplatte ist, das heißt dass in diesem Fall eine Nervenbahnblockade gleich einer gestörten chronisch aktivierten motorischen Endplatte ist. Es ist aber auch möglich, dass die Nervenbahnblockade in einem anderen Bestandteil des gestörten Nervs vorliegt, das heißt, dass die Lokalisierung der Nervenbahnblockade räumlich nicht identisch mit der chronisch aktivierten motorischen Endplatte ist. Das bedeutet insbesondere auch, dass die Nervenbahnblockade auch andere physiologische Prozesse des betroffenen Nervs stören kann und nicht ausschließlich auf Prozesse in der motorischen Endplatte begrenzt ist. Die Nervenbahnblockade kann insbesondere auch genau dort lokalisiert sein, wo ein druckempfindlicher Triggerpunkt im Muskel vorliegt, der bei Ausübung von manuellem Druck Schmerzen in andere, teilweise weit entfernte, Bereiche des Körpers ausstrahlt. Der Bereich in den die Schmerzen ausstrahlen kann aber auch regional sein, das heißt nah an der Stelle an der die Druckausübung stattfindet. Die Nervenbahnblockade ist dabei als neurologische Komponente zu verstehen, die an der Ausbildung von Triggerpunkten beteiligt ist. Daneben ist auch eine muskuläre Komponente beteiligt.

Als Bereich des Nervus trigeminus in dem die erfindungsgemäße Zusammensetzung verwendet wird, indem die Zusammensetzung in diesem Bereich auf die Haut aufgetragen wird, ist der Bereich auf der Haut zu verstehen, der sich im Wesentlichen oberhalb der Verlängerung einer gedachten, horizontalen Linie befindet, die gebildet wird, indem sie durch die wesentliche Mitte der Ohrmuschel in Bezug auf die z-Achse verläuft, wobei sich der Bereich im Wesentlichen vor der Ohrmuschel, also zur Richtung des Gesichts hin, befindet, insbesondere exakt dort, wo in genau diesem Bereich ein Tenderpunkt, also eine tastbare, knotenartige Verdickung im Bindegewebe vorliegt, der bei Ausübung eines manuellen Drucks einen Schmerzreiz auslöst, insbesondere also genau dort wo in diesem Bereich eine Faszienanhaftung des Musculus occipitofrontalis an der Knochenhaut unter Beteiligung des Nervus trigeminus vorliegt.

Als Bereich des Nervus facialis in dem die erfindungsgemäße Zusammensetzung verwendet wird, indem die Zusammensetzung in diesem Bereich auf die Haut aufgetragen wird, ist der Bereich auf der Haut zu verstehen, der sich im Wesentlichen unterhalb der Verlängerung einer gedachten Linie befindet, die gebildet wird, indem sie vertikal durch den Mittelpunkt der Öffnung des äußeren Gehörgangs verläuft, wobei sich der Bereich im Wesentlichen direkt unterhalb der Öffnung des äußeren Gehörgangs, insbesondere unter der Ohrmuschel, befindet, insbesondere exakt dort, wo in genau diesem Bereich ein Tenderpunkt, also eine tastbare, knotenartige Verdickung im Bindegewebe vorliegt, der bei Ausübung eines manuellen Drucks einen Schmerzreiz auslöst, insbesondere also genau dort wo in diesem Bereich eine Faszienanhaftung des Musculus occipitofrontalis an der Knochenhaut unter Beteiligung des Nervus trigeminus vorliegt.

Als Bereich des Nervus medianus in dem die erfindungsgemäße Zusammensetzung verwendet wird, indem die Zusammensetzung in diesem Bereich auf die Haut aufgetragen wird, ist der Bereich auf der Haut zu verstehen, der sich im Wesentlichen unterhalb der Verlängerung einer gedachten Linie befindet, die gebildet wird indem sie, bei ausgestreckter, supinierter Hand, durch die wesentliche Mitte des Zeigefingers in horizontaler Richtung, in Richtung des Arms verläuft, wobei sich der Bereich im Wesentlichen direkt oberhalb (proximal) der Handfläche im Bereich des Handgelenks befindet, insbesondere exakt dort, wo in genau diesem Bereich ein Tenderpunkt, also eine tastbare, knotenartige Verdickung im Bindegewebe vorliegt, der bei Ausübung eines manuellen Drucks einen Schmerzreiz auslöst, insbesondere also genau dort wo in diesem Bereich eine Faszienanhaftung des Musculus flexor pollicis longus an der Knochenhaut des Os radius unter Beteiligung des Nervus medianus vorliegt.

Als Bereich des Nervus ischiadicus in dem die erfindungsgemäße Zusammensetzung verwendet wird, indem die Zusammensetzung in diesem Bereich auf die Haut aufgetragen wird, ist der Bereich auf der Haut zu verstehen, der sich im Wesentlichen unter der Verlängerung einer gedachten Linie befindet, die gebildet wird indem sie, bei ausgestrecktem, neutral gestelltem Fuß, durch die wesentliche Mitte des Großen Zehs (Digitus Pedis I) in vertikaler Richtung auf der Fußsohle, in Richtung der Ferse verläuft, wobei sich der Bereich im Wesentlichen direkt dort befindet, wo die gedachte Linie den Fußballen schneidet, insbesondere exakt dort, wo in genau diesem Bereich ein Tenderpunkt, also eine tastbare, knotenartige Verdickung im Bindegewebe vorliegt, der bei Ausübung eines manuellen Drucks einen Schmerzreiz auslöst, insbesondere also genau dort wo in diesem Bereich eine Faszienanhaftung des Musculus flexor hallucis longus an der Knochenhaut des Os sesamoidale unter Beteiligung des Nervus ischiadicus vorliegt.

Es wurde erkannt, dass in einer als besonders unangenehm beschriebenen Form die Ausbildung von Tenderpunkten im Bereich des Schädels zu Druckausgleichsstörungen des Ohres führt. Interessanterweise sind ebenfalls Tenderpunkte im Bereich des inneren Handgelenks, also genau dort wo in diesem Bereich eine Faszienanhaftung des Musculus flexor pollicis longus an der Knochenhaut des Os radius unter Beteiligung des Nervus medianus vorliegt, und im Bereich des Fußballens, also genau dort wo in diesem Bereich eine Faszienanhaftung des Musculus flexor hallucis longus an der Knochenhaut des Os sesamoidale unter Beteiligung des Nervus ischiadicus vorliegt, an diesem Prozess beteiligt. Ursache dieser Beschwerden ist die gestörte dauerhafte Aktivierung des Musculus tensor tympanii durch einen oder mehrere Tenderpunkte. Dabei spannt der chronisch aktivierte Muskel das Trommelfell derart nach innen, dass ein freies Schwingen nicht mehr möglich ist und es zu einem Druckunterschied zwischen Mittel- und Außenohr kommt. Durch die Straffung des Trommelfells kann dieser nicht mehr auf physiologische Weise durch die Eustachische Röhre, die vom Mittelohr in den Rachenraum verläuft, ausgeglichen werden. Der Druckunterschied zwischen Mittel- und Außenohr geht mit einer verringerten Hörleistung, einem unangenehmen Druckgefühl, Gleichgewichtsstörungen, Ohrgeräuschen und Schmerzen einher. Dies kann auch zu Folgeschäden, wie Haltungsschäden und bei dauerhafter Störung zu orthopädischen Beeinträchtigungen, wie Muskelverringerung und einseitig verkürzten Beinen, einseitiger Skoliose, Schulter- und Beckenschiefstand, sowie dem einseitigen Einzug von Zungenbein und Kiefergelenk führen.

Es wurde außerdem beobachtet, dass die Beteiligung des sympathischen und parasympathischen Nervensystems an der Ausbildung von Tenderpunkten und Triggerpunkten beteiligt sein kann und dadurch eine Störung dieser hervorrufen kann. So führen Störungen im als aktivierend auf den Körper wirkenden sympathischen Nervensystem dazu, dass der Betroffenen eine Rastlosigkeit empfindet und nur schwer zur Ruhe kommen kann. Die Störung des als beruhigend auf den Körper wirkenden parasympathischen Nervensystems führt zu starker Müdigkeit und Teilnahmslosigkeit.

Mit der erfindungsgemäßen Verwendung der Zusammensetzung zur Auflösung von Faszienverkürzungen, indem die Zusammensetzung im Bereich des Nervus trigeminus, des Nervus facialis, des Nervus medianus, des Nervus ischiadicus aufgetragen wird, können damit auch nicht unmittelbar mit der Eustachischen Röhre zusammenhängende Fehlentwicklungen entgegengewirkt werden.

In einer Ausführungsform ist die erfindungsgemäße Zusammensetzung eine, die verwendet wird, in dem die Zusammensetzung auf eine Kombination aus mindestens zwei dieser Bereiche auf die Haut aufgetragen wird. In einer anderen Ausführungsform wird die erfindungsgemäße Zusammensetzung verwendet, in dem die Zusammensetzung auf eine Kombination aus drei dieser Bereiche auf die Haut aufgetragen wird. Es ist auch möglich die erfindungsgemäße Zusammensetzung zu verwenden, indem die Zusammensetzung auf eine Kombination aller vier Bereiche auf die Haut aufgetragen wird. Es ist ausdrücklich auch möglich die erfindungsgemäße Zusammensetzung zu verwenden, indem die Zusammensetzung nur auf einen der Bereiche auf die Haut aufgetragen wird.

Die erfindungsgemäße Zusammensetzung muss nicht so verwendet werden, dass die Zusammensetzung direkt auf einen Nerv aufgetragen wird. In jedem Fall ist die erfindungsgemäße Zusammensetzung daher eine, die verwendet wird, indem die Zusammensetzung im Bereich eines entsprechenden Nervs auf die Haut aufgetragen wird und durch die Hautschichten den darunter liegenden Nerv erreicht.

Insbesondere sind die Bereiche der entsprechenden Nerven, in denen die erfindungsgemäße Zusammensetzung durch Auftragen der Zusammensetzung in diesem Bereich verwendet wird, als solche Bereiche zu verstehen in denen die entsprechenden Nerven nahe unter der Haut verlaufen.

Dabei sind die Form und die Fläche des Bereichs an sich, also unabhängig des betreffenden Nervs in dessen Bereich die erfindungsgemäße Zusammensetzung auf die Haut aufgetragen wird, unerheblich für die Wirksamkeit der erfindungsgemäßen Zusammensetzung. Das bedeutet, dass die Wahl der Fläche und der Form des Bereichs auf der Haut, auf den die erfindungsgemäße Zusammensetzung aufgetragen wird, keinen Einfluss auf die Wirksamkeit der erfindungsgemäßen Zusammensetzung hat. Das bedeutet wiederum, dass die Form und die Fläche des Bereichs auf der Haut, auf den die erfindungsgemäße Zusammensetzung aufgetragen werden soll, frei gewählt werden kann. Es soll dabei jedoch stets eine ausreichende Menge der erfindungsgemäßen Zusammensetzung auf die Haut aufgetragen werden, um diese sichtbar und vollständig im gesamten ausgewählten Bereich zu benetzen.

In einer bevorzugten Ausführungsform ist die erfindungsgemäße Zusammensetzung eine, die im Bereich des Nervus trigeminus, des Nervus facialis, des Nervus medianus, des Nervus ischiadicus oder auf eine Kombination aus mindestens zwei dieser Bereiche auf die Haut aufgetragen wird, wobei der Bereich dabei eine kreisförmige Form mit einem Durchmesser von 3 cm, bevorzugterweise von 2 cm und noch bevorzugterweise von 1,5 cm, aufweist.

Es ist insbesondere nicht so zu verstehen, dass die erfindungsgemäße Zusammensetzung nur auf einen Bereich auf die Haut aufgetragen werden kann, der ausschließlich eine kreisförmige Form aufweist. Die Form des Bereichs kann vielmehr ebenfalls rechteckig, trapezförmig oder elliptisch sein. Es ist auch möglich die erfindungsgemäße Zusammensetzung in einem Bereich auf die Haut aufzutragen, der keine vorher definierte, also eine zufällige, Form aufweist.

Es wurde erkannt, dass es besonders vorteilhaft ist, die erfindungsgemäße Zusammensetzung, die einen Träger und Natriumchlorid enthält, zur Verwendung zur Auflösung von Faszienverkürzungen zu verwenden, indem die Zusammensetzung im Bereich ausgewählt aus dem Bereich des Nervus trigeminus, des Nervus facialis, des Nervus medianus, des Nervus ischiadicus oder auf eine Kombination aus mindestens zwei dieser Bereiche auf die Haut aufgetragen wird, wenn die Faszienverkürzung auf der rechten Körperhälfte vorliegt. Dabei hat es sich außerdem als besonders vorteilhaft erwiesen, die Zusammensetzung zur Verwendung zur Auflösung von Faszienverkürzungen zu verwenden, indem die Zusammensetzung zusätzlich im Bereich des Musculus flexor capri ulnaris und des Musculus flexor digitorium longus auf der linken Körperseite oder auf eine Kombination beider Bereiche zur Unterstützung der Verwendung der erfindungsgemäßen Zusammensetzung auf die Haut aufgetragen wird.

Außerdem wurde erkannt, dass es besonders vorteilhaft ist, die erfindungsgemäße Zusammensetzung, die einen Träger und Kaliumchlorid enthält, zur Verwendung zur Auflösung von Faszienverkürzungen zu verwenden, indem die Zusammensetzung im Bereich ausgewählt aus dem Bereich des Nervus trigeminus , des Nervus facialis, des Nervus medianus, des Nervus ischiadicus oder auf eine Kombination aus mindestens zwei dieser Bereiche auf die Haut aufgetragen wird, wenn die Faszienverkürzung auf der linken Körperhälfte vorliegt. Dabei hat es sich außerdem als besonders vorteilhaft erwiesen, die Zusammensetzung zur Verwendung zur Auflösung von Faszienverkürzungen zu verwenden, indem die Zusammensetzung zusätzlich im Bereich des Musculus flexor capri ulnaris und des Musculus flexor digitorium longus auf der rechten Körperseite oder auf eine Kombination beider Bereiche auf die Haut aufgetragen wird.

Die Wirksamkeit der erfindungsgemäßen Zusammensetzung lässt sich aus der physikalischen Überlegung ableiten, dass die mit der Faszienverkürzung assoziierte Nervenbahnblockade als neurologische Komponente der Triggerpunkt-Formation auf zellulärer Ebene mit Nervenzellen im Zusammenhang steht. Zur Aufrechterhaltung und Wiederherstellung des Ruhepotentials arbeiten Natrium-Kalium-Pumpen in der Plasmamembran von Nervenzellen unter ATP-Verbrauch. Erfindungsgemäß wird dieser elektrochemische Wirkmechanismus ausgenutzt, indem ein gestörtes Verhältnis der intra- und extrazellulären Konzentration von Natrium- und Kaliumionen ausgelöst durch gestörte Natrium-Kalium-Pumpen bei der eine Elektrolytverschiebung von Kalium oder Natrium vorliegt, durch Auftragen von Natriumchlorid bzw. Kaliumchlorid wieder ins Gleichgewicht gebracht wird. Dabei wird ausgenutzt, dass die Hautbarriere für Kationen durchlässig ist.

Als Bereich des Musculus flexor capri ulnaris in dem die erfindungsgemäße Zusammensetzung verwendet wird, indem die Zusammensetzung in diesem Bereich auf die Haut aufgetragen wird, ist der Bereich auf der Haut zu verstehen, der sich im Wesentlichen auf der dorsalen Seite der ausgestreckten Hand zwischen dem Ossa metacarpalia IV und dem Ossa metacarpalia V vom Handrücken aus gesehen, insbesondere genau dort, wo in diesem Bereich der Musculus flexor capri ulnaris verläuft, befindet.

Als Bereich des Musculus flexor digitorium longus in dem die erfindungsgemäße Zusammensetzung verwendet wird, indem die Zusammensetzung in diesem Bereich auf die Haut aufgetragen wird, ist der Bereich auf der Haut zu verstehen, der sich im Wesentlichen auf der dorsalen Seite des neutral gestellten Fußes zwischen dem Ossa metatarsi IV und dem Ossa metatarsi V vom Fußrücken aus gesehen, insbesondere genau dort, wo in diesem Bereich der Musculus flexor digitorium longus verläuft, befindet.

Dabei wurde erkannt, dass es besonders vorteilhaft ist, die erfindungsgemäße Zusammensetzung, zur Verwendung zur Auflösung von Faszienverkürzungen zu verwenden, indem die Zusammensetzung im Bereich ausgewählt aus dem Bereich des Nervus trigeminus, des Nervus facialis, des Nervus medianus, des Nervus ischiadicus oder auf eine Kombination aus mindestens zwei dieser Bereiche auf die Haut aufgetragen wird, zur Auflösung von Faszienverkürzungen zu verwenden, da so die resultierende Körperasymmetrie begradigt wird, die Körperbewegungen wieder gleichmäßig ablaufen und die Triggerpunkte in der Skelettmuskulatur automatisch inaktiviert werden.

Es ist besonders vorteilhaft, dass die erfindungsgemäße Zusammensetzung derart verwendet wird, dass früh in die Entstehungsmechanismen von Triggerpunkten eingegriffen wird, sodass die eigentliche Ursache behoben wird, nämlich die Formation von Tenderpunkten durch Faszienverkürzungen. Es ist daher vorteilhaft, dass langwierige und schmerzhafte Massageanwendungen zur manuellen Auflösung der Triggerpunkte auf muskulär Ebene nicht mehr notwendig sind. Die Massagebehandlung, die dem Fachmann aus dem Stand der Technik bereits bekannt ist, kann als unterstützende Maßnahme effektiver und mit geringerer Intensität angewendet werden. Vorteilhaft ist also, dass die Behandlung von Triggerpunkten weniger schmerzhaft und langwierig ist und die damit verbundene, erhöhte Akzeptanz beim Verwender.

Da die erfindungsgemäße Zusammensetzung, derart verwendet wird, dass früh und ganzheitlich in die Entstehungsmechanismen von Triggerpunkten auf neurologischer und faszialer Ebene eingriffen wird, liegt außerdem ein geringeres Risiko vor, dass sich soeben aufgelöste Triggerpunkte erneut ausbilden. Dies ist insbesondere gegenüber dem Stand der Technik von Vorteil, der darauf abzielt Triggerpunkte auf muskulärer Ebene aufzulösen. Dabei werden ausschließlich Symptome behandelt und die eigentlichen Ursachen bleiben bestehen, sodass ein hohes Rückfallrisiko vorliegt.

Die erfindungsgemäße Zusammensetzung wird verwendet, indem die Zusammensetzung im Bereich der betroffenen Nerven auf die Haut aufgetragen wird. Es findet dabei keine Verletzung der Haut statt. Dies hat den Vorteil, dass die Verwendung der erfindungsgemäßen Zusammensetzung mit keinem erhöhten Infektionsrisiko verbunden ist, wie es etwa bei der Nadelbehandlung von Triggerpunkten, auch in Kombination mit lokalwirkenden Anästhetika, durchaus der Fall ist.

In einer Ausführungsform enthält die erfindungsgemäße Zusammensetzung Acetylcholin.

Es hat sich gezeigt, dass Acetylcholin, das als Neurotransmitter im Bereich des synaptischen Spalts essentiell an der physiologischen Funktion von Nervenzellen beteiligt ist, die Wirksamkeit der erfindungsgemäßen Zusammensetzung vorteilhafterweise erhöht. Dies könnte daran liegen, dass die Entstehung von Tenderpunkten auf neurologischer Ebene mit einem gestörten Acetylcholin-Stoffwechsel zusammenhängt und die Zugabe von Acetylcholin diese Störung aufhebt.

In einer Ausführungsform enthält die erfindungsgemäße Zusammensetzung Phosphat. Dabei kann Phosphat der erfindungsgemäßen Zusammensetzung in Form eines anorganischen oder organischen Salzes, die jeweils dermatologisch vertretbare Eigenschaften in Bezug auf eine gute Hautverträglichkeit aufweisen müssen, zugegeben werden. Darüber hinaus kann Phosphat auch in einem komplexen Stoffgemisch enthalten sein, etwa einem Pflanzenextrakt, das der erfindungsgemäßen Zusammensetzung zugegeben wird.

Es hat sich herausgestellt, dass Phosphat die Wirksamkeit der erfindungsgemäßen Zusammensetzung vorteilhafterweise erhöht. Dies könnte daran liegen, dass die Entstehung von Tenderpunkten und Triggerpunkten auf muskulärer Ebene mit einer Dauerkontraktion der Muskeln und einem stark erhöhten Energiebedarf zusammenhängt. Phosphat ist essentieller Bestandteil von ATP, das in der Muskelzelle als Energiespeicher genutzt wird. Es besteht die Möglichkeit, dass die Zugabe von Phosphat den ATP-Stoffwechsel positiv beeinflusst.

In einer Ausführungsform enthält die erfindungsgemäße Zusammensetzung einen Extrakt aus Brennnessel, der Acetylcholin und Phosphat enthält.

Es hat sich als vorteilhaft herausgestellt einen Extrakt aus Brennnessel zu verwenden, da dieser natürlicherweise bereits hohe Mengen der beiden Wirkstoffe enthält. Die Verwendung eines Extrakts aus Brennnessel ist dem Fachmann bekannt, da die Brennnessel schon seit langem als Heilpflanze mit positiven Eigenschaften verwendet wird.

In einer bevorzugten Ausführungsform ist der Extrakt aus Brennnessel einer, der mittels öliger Extraktion hergestellt wurde, noch bevorzugter einer, für den Olivenöl als Extraktionsmittel für die ölige Extraktion verwendet wurde. Es besteht auch die Möglichkeit, dass der Brennnesselextrakt mittels eines anderen Extraktionsverfahren hergestellt wurde. Mögliche Extraktionsverfahren sind wässrige, alkoholische, glykolische oder gemischte Extraktion. Dabei kann die wässrige Extraktion als kochender Aufguss, als Abkochung oder als Kaltauszug durchgeführt worden sein. Bei der Auswahl der verwendeten Extraktionsmittel ist jedoch stehts auf dermatologisch annehmbare Eigenschaften in Bezug auf eine gute Hautverträglichkeit zu achten.

Es hat sich gezeigt, dass die Kombination der beiden Komponenten Acetylcholin und Phosphat die Wirksamkeit der erfindungsgemäßen Zusammensatzung dabei über ein Maß hinaus steigert, das bei der Verwendung jeweils einer Komponente nicht erreicht wird. Diese vorteilhafte Synergie von Acetylcholin und Phosphat kann darin begründet sein, dass Acetylcholin auf neurologischer Ebene und Phosphat auf muskulärer Ebene eine positive Wirkung bei der Auflösung von Faszienverkürzungen entfaltet. Die vorteilhafte Kombination der beiden Komponenten schließt jedoch explizit nicht aus, die Komponenten jeweils einzeln in einer individuellen Ausführungsform der erfindungsgemäßen Zusammensetzung zu verwenden.

In einer Ausführungsform enthält die erfindungsgemäße Zusammensetzung mindestens eine dermatologisch annehmbare Trägersubstanz als Träger. Es ist auch möglich, dass die erfindungsgemäße Zusammensetzung mehr als eine dermatologisch annehmbare Trägersubstanz enthält. Eine solche Kombination aus dermatologisch vertretbaren Trägersubstanzen kann dazu führen, dass es sich bei der erfindungsgemäßen Zusammensatzung um eine Creme, eine Salbe oder eine Lotion handelt. Es ist jedoch nicht ausgeschlossen, dass die erfindungsgemäße Zusammensetzung nur einen einzelnen dermatologisch annehmbaren Träger beinhaltet.

Vorteilhaft an dieser Zusammensetzung ist die hohe Hautverträglichkeit von dermatologisch annehmbaren Trägersubstanzen. So werden Unverträglichkeiten und allergische Reaktionen vermieden. Es ist außerdem von Vorteil, dass dermatologisch annehmbare Trägersubstanzen in der Regel bevorzugte Verarbeitungs- und Verwendungseigenschaften aufweisen. Es ist dabei von Vorteil, dass der dermatologisch annehmbare Träger insbesondere derart ausgewählt wird, die anderen Komponenten der erfindungsgemäßen Zusammensetzung schnell und komplett aufzunehmen, nach der Verarbeitung eine beständige und homogene Struktur aufzuweisen und die einfache und großflächige Auftragung der erfindungsgemäßen Zusammensetzung im Bereich des Nervus trigeminus, des Nervus facialis, des Nervus medianus, Nervus ischiadicus oder auf eine Kombination aus mindestens zwei dieser Bereiche auf die Haut zu ermöglichen.

Die hier gemeinten dermatologisch annehmbare Trägersubstanzen sind dem Fachmann lange bekannt.

Bei den dermatologisch annehmbaren Trägersubstanzen kann es sich insbesondere um eine, ausgewählt aus der folgenden Liste handeln:
a. hydrophoben Salben, einschließlich der Carbogele, insbesondere weißer Vaseline, der Lipogele, insbesondere Wachssalbe, und der Silikongele;
b. wasseraufnehmenden Salben, einschließlich der Absorptionsbasen Wasser in Öl, insbesondere Wollwachsalkoholsalbe, und der Absorptionsbasen Öl in Wasser;
c. hydrophilen Salben, einschließlich der festen und flüssigen Macrogole;
d. hydrophoben Cremes, einschließlich Wollwachsalkoholcreme DAB;
e. hydrophile Cremes;
f. hydrophoben Gele;
g. hydrophile Gele;
h. hydrophobe Pasten;
i. hydrophile Pasten.

In einer bevorzugten Ausführungsform der erfindungsgemäßen Zusammensetzung ist das Gewichtsverhältnis von entweder Natriumchlorid oder Kaliumchlorid zu Brennnesselextrakt zu mindestens einer dermatologisch annehmbaren Trägersubstanz eines, das in einem Bereich von 1 bis 10 Gewichtsprozenten Natriumchlorid oder Kaliumchlorid und 1 bis 28 Gewichtsprozenten Brennnesselextrakt und 1 bis 62 Gewichtsprozenten mindestens einer dermatologisch annehmbaren Trägersubstanz liegt.

Es hat sich gezeigt, dass eine erfindungsgemäße Zusammensetzung die ein entsprechendes Gewichtsverhältnis aufweist, eine besonders hohe Wirksamkeit aufweist. Dies kann daran liegen, dass bei diesem Gewichtsverhältnis die einzelnen Komponenten ihre optimale Wirksamkeit entfalten und so besonders vorteilhafte Synergien entstehen.

In einer Ausführungsform der erfindungsgemäßen Zusammensetzung ist die mindestens eine dermatologisch annehmbare Trägersubstanz gereinigtes Wasser.

In einer besonders bevorzugten Ausführungsform ist die erfindungsgemäße Zusammensetzung eine, die entweder Natriumchlorid oder Kaliumchlorid mit einem Anteil von jeweils 10 Gewichtsprozent und Brennnesselextrakt, mit einem Anteil von 28 Gewichtsprozent, sowie gelbes Wachs mit einem Anteil von 3 bis 4 Gewichtsprozent, Wollwachsalkoholsalbe DAB mit einem Anteil 18 bis 20 Gewichtsprozent, sowie gereinigtes Wasser mit einem Anteil von 38 bis 42 Gewichtsprozent beinhaltet. Die Angaben sind so zu verstehen, dass eine übliche Toleranz möglich ist, z. B. eine Abweichung der genannten Gewichtsprozent um ±10%.

Besonders vorteilhaft an dieser erfindungsgemäßen Zusammensetzung ist eine hohe Hautverträglichkeit bei einer gleichzeitig hohen Wirksamkeit.

Das Auftragen der erfindungsgemäßen Zusammensetzung sollte unter Druck, bevorzugterweise in kreisenden Bewegungen von distal nach proximal in Faserrichtung, auf die Bereiche der betroffenen Tenderpoints und Muskelansätze erfolgen, um die Faszienanhaftung und Muskelanhaftung an der Knochenhaut zu lösen. Dies kann mit einem Applikator mit einer Druck-Auftragungseinheit zum Auftragen der Zusammensetzung unter mechanischem Druck auf die Haut erreicht werden, bei dem die Auftragungseinheit zum Auftragen der Zusammensetzung auf die Haut ein Ultraschallapplikator mit einem zu Ultraschallsschwingungen anregbarem Ultraschallkopf ist. Der Ultraschallapplikator ist zur Aufmodulation einer Frequenz von wahlweise 20 Hertz oder 50 Hertz ausgebildet. Damit kann der Ultraschallapplikator zum Auftragen der Zusammensetzung auf den Ultraschallkopf verwendet werden, um wahlweise die Natriumchlorid enthaltende Zusammensetzung mit Ultraschall und einer aufmodulierten Frequenz von 50 Hertz oder die Kaliumchlorid enthaltende Zusammensetzung mit Ultraschall und einer aufmodulierten Frequenz von 20 Hertz auf den ausgewählten Bereich der Tenderpoints und Muskelanhaftungen zu applizieren.

Der Ultraschallapplikator kann zum Auftragen der erfindungsgemäßen Zusammensetzung auf den Ultraschallkopf und Applikation auf dem jeweils zu behandelnden Triggerpunkt mit dem Ultraschallkopf verwendet werden, um wahlweise die Natriumchlorid enthaltende erfindungsgemäße Zusammensetzung mit Ultraschall und einer aufmodulierten Frequenz von 50 Hertz oder die Kaliumchlorid enthaltende erfindungsgemäße Zusammensetzung mit Ultraschall und einer aufmodulierten Frequenz von 20 Hertz jeweils im Bereich ausgewählt aus dem Bereich des Nervus trigeminus, des Nervus facialis, des Nervus medianus, des Nervus ischiadicus oder auf eine Kombination aus mindestens zwei dieser Bereiche auf die Haut aufzutragen.

So kann der Ultraschallapplikator verwendet werden, um wahlweise die Natriumchlorid enthaltene Zusammensetzung mit einer aufmodulierten Frequenz von 50 Hz für den Nervus sympaticus bzw. den zugehörigen Tenderpunkt oberhalb der rechten Augenbraue oder die Kaliumchlorid enthaltene Zusammensetzung mit einer aufmodulierten Frequenz von 20 Hz für den Vagusnerv (Nervus vagus) bzw. den zugehörigen Tenderpunkt oberhalb der linken Augenbraue. Die Tenderpunkte, in denen das Auftragen der Zusammensetzung unter Druck erfolgt, sind in der Regel als geschwollene, etwa erbsengroße druckempfindliche Verdickungen ertastbar.

Die Verwendung der Zusammensetzung durch Auftragen der erfindungsgemäßen Zusammensetzung unter Druck auf die Bereiche der betroffenen Tenderpunkte und Muskelansätze kann auch mit einem Applikator erfolgen, der die erfindungsgemäße Zusammensetzung, die entweder Natriumchlorid oder Kaliumchlorid beinhaltet, enthält, oder derart gestaltet ist, dass sich die erfindungsgemäße Zusammensetzung mit einer Druck-Auftragseinheit auftragen lässt. Dabei weist der erfindungsgemäße Applikator eine Auftragungseinheit auf, mit der die erfindungsgemäße Zusammensetzung auf die Haut aufgetragen wird.

In einer Ausführungsform ist der erfindungsgemäße Applikator einer, der als Roll-On Applikator mit einem Behälter, der die erfindungsgemäße Zusammensetzung, die entweder Natriumchlorid oder Kaliumchlorid beinhaltet, enthält, gestaltet ist. Der Behälter weist einen Rollkopf mit einer drehbar gelagerten Auftragerolle auf, der eine Öffnung des Behälters verschließt, und der die Auftragung der erfindungsgemäßen Zusammensetzung auf die Haut ermöglicht.

Vorteilhaft an diesem erfindungsgemäßen Applikator ist, dass so eine einfache Verwendung durch den Anwender möglich ist und kein Fachmann zur Anwendung benötigt wird. Die unkomplizierte Verwendung durch den Anwender erhöht außerdem die Akzeptanz und damit die Wirksamkeit der erfindungsgemäßen Zusammensetzung durch eine sachdienliche Frequenz der Verwendung.

In einer Ausführungsform ist die drehbar gelagerte Auftragerolle des Rollkopfes des erfindungsgemäßen Applikators, derart strukturiert, dass der Rollkopf des erfindungsgemäßen Applikators Noppen, oder ähnliche Vorsprünge, aufweist, wobei diese Noppen beim Auftragen der erfindungsgemäßen Zusammensetzung auf die Haut eine massierende Wirkung auf diese haben.

Vorteilhaft an dieser Ausführungsform ist, dass so die erfindungsgemäße Zusammensetzung schneller in die Bereiche, in denen die Zusammensetzung auf die Haut aufgetragen wird, eindringen kann und so schneller die volle Wirksamkeit der Zusammensetzung entfaltet wird. Diese Ausführungsform hat den weiteren Vorteil, dass die massierende Wirkung des entsprechend gestalteten Rollkopfs des erfindungsgemäßen Applikators die Durchblutung der Haut in dem Bereich auf den die erfindungsgemäße Zusammensetzung aufgetragen wurde, anregt. Es hat sich gezeigt, dass die erhöhte Durchblutung, angeregt durch den entsprechend strukturierten Rollkopf des erfindungsgemäßen Applikators, die Wirksamkeit der erfindungsgemäßen Zusammensetzung, die mit dem erfindungsgemäßen Applikator auf die Haut aufgetragen wurde, erhöht.

Es wurde erkannt, dass es besonders vorteilhaft ist, die einen Träger und Natriumchlorid enthaltende Zusammensetzung zur Auflösung von Faszienverkürzungen zu verwenden, indem die Zusammensetzung im Bereich des Nervus trigeminus, des Nervus facialis, des Nervus medianus, des Nervus ischiadicus oder auf eine Kombination aus mindestens zwei dieser Bereiche durch einen Applikator unter Druck aufzutragen. Besonders geeignet ist die Verwendung eines Ultraschallapplikators, der zur Aufmodulation einer Frequenz von 50 Hertz ausgebildet ist. Der Ultraschallapplikator weist einen zum Auftragen der erfindungsgemäßen Zusammensetzung auf die Haut ausgebildeten und zu Ultraschallschwingungen anregbaren Ultraschallkopf auf, um die Natriumchlorid als Wirkstoff enthaltende Zusammensetzung unter Applikation von Ultraschall mit einer aufmodulierten Frequenz von 50 Hertz auf die Haut aufzutragen, wenn eine Faszienverkürzung auf der rechten Körperhälfte und eine Muskelverkürzung auf der linken Körperseite vorliegt.

Außerdem wurde erkannt, dass es besonders vorteilhaft ist, die einen Träger und Kaliumchlorid enthaltende Zusammensetzung zur Verwendung zur Auflösung von Faszienverkürzungen zu verwenden, indem die Zusammensetzung im Bereich ausgewählt aus dem Bereich des Nervus trigeminus, des Nervus facialis, des Nervus medianus, des Nervus ischiadicus oder auf eine Kombination aus mindestens zwei dieser Bereiche durch einen Applikator unter Druck aufzutragen. Besonders geeignet ist die Verwendung eines Ultraschallapplikators, der zur Aufmodulation einer Frequenz von 20 Hertz ausgebildet ist und einen zum Auftragen der erfindungsgemäßen Zusammensetzung auf die Haut ausgebildeten und zu Ultraschallschwingungen anregbaren Ultraschallkopf aufweist, um die Kaliumchlorid als Wirkstoff enthaltende Zusammensetzung unter Applikation von Ultraschall mit einer aufmodulierten Frequenz von 20 Hertz auf die Haut aufzutragen, wenn die Faszienverkürzung auf der linken Körperhälfte und eine Muskelverkürzung auf der rechten Körperseite vorliegt.

Die Aufgabe wird auch durch die Verwendung einer solchen Zusammensetzung für die Auflösung von Faszienverkürzungen durch Auftragen mit einem Set gelöst, das aus einem ersten Applikator, der die Natriumchlorid enthaltende, erfindungsgemäße Zusammensetzung beinhaltet und zum Auftragen der Natriumchlorid enthaltenden erfindungsgemäßen Zusammensetzung auf die Haut ausgebildet ist, zur Verwendung für die Auflösung von Faszienverkürzungen, indem die erfindungsgemäße Zusammensetzung im Bereich des Nervus trigeminus, des Nervus facialis, des Nervus medianus, des Nervus ischiadicus oder auf eine Kombination aus mindestens zwei dieser Bereiche auf die Haut aufgetragen wird. Ferner besteht das erfindungsgemäße Set aus einem zweiten erfindungsgemäßen Applikator, der die Kaliumchlorid enthaltende, erfindungsgemäße Zusammensetzung beinhaltet und zum Auftragen der Kaliumchlorid enthaltenden erfindungsgemäßen Zusammensetzung auf die Haut ausgebildet ist, zur Verwendung für die Auflösung von Faszienverkürzungen, indem die erfindungsgemäße Zusammensetzung im Bereich des Nervus trigeminus, des Nervus facialis, des Nervus medianus, des Nervus ischiadicus oder auf eine Kombination aus mindestens zwei dieser Bereiche auf die Haut aufgetragen wird.

### Ausführungsbeispiele

Die Erfindung wird nachfolgend anhand der beigefügten Zeichnungen beispielhaft mit Ausführungsbeispielen beschrieben. Es zeigen:
- Fig. 1: - Skizze eines Ultraschallapplikators mit zwei wahlweise zu verwendenden cremeartigen Zusammensetzungen;
- Fig. 2: - Skizze eines Sets mit zwei Applikatoren in Form eines Roll-On-Stiftes;
- Fig. 3: - Skizze der rechten und linken Nervenbahnen.

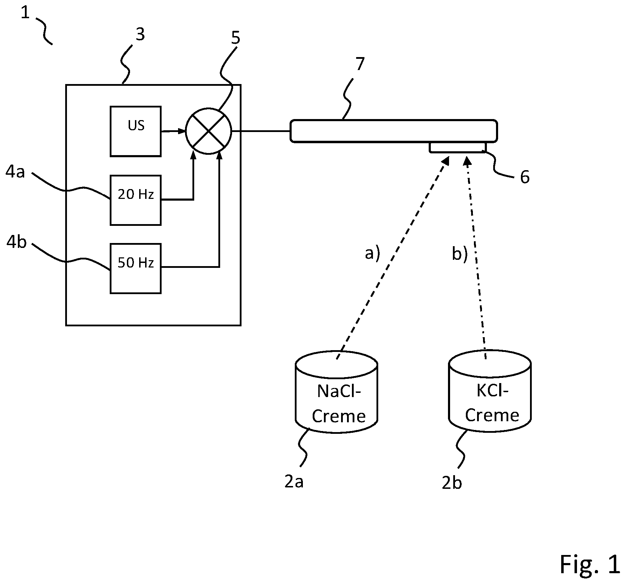

Figur 1 zeigt eine Skizze eines Ultraschallapplikators 1 mit zwei wahlweise zu verwendenden cremeartigen Zusammensetzungen in zwei Behältern 2a, 2b. Die im Behälter 2a abgefüllte Zusammensetzung enthält als Wirkstoff Natriumchlorid NaCl in einem dermatologisch annehmbaren Träger in Form einer Creme. Die in dem anderen Behälter 2b abgefüllte Zusammensetzung enthält als Wirkstoff Kaliumchlorid KCl in einem dermatologisch annehmbaren Träger in Form einer Creme

Der Ultraschallapplikator 1 hat eine Ansteuerungseinheit 3 mit einem UltraschallFrequenzgenerator US und mindestens einem Niederfrequenzgenerator 4a, 4b zur Erzeugung einer Modulationsfrequenz von wahlweise 20 Hz 4a oder 50 Hz 4b. Die ausgewählte Niederfrequenz von entweder 20 Hz oder 50 Hz wird auf die vom UltraschallFrequenzgenerator US erzeugte Hochfrequenz zur Anregung eines Ultraschallaktuators 6 mit einem Mischer 5 gemischt.

Optional ist denkbar, die Niederfrequenz als elektrisches Spannungssignal zusätzlich zu dem zu mechanischen Ultraschallschwingungen angeregten Ultraschallaktuator 6 an einen elektrisch leitenden Bereich des Ultraschallaktuators 6 oder eines diesen umgebenden Elements zu leiten.

Der Ultraschallaktuator 6 ist am Kopf eines Handgehäuses 7 angeordnet und mit einer elektrischen Leitung mit der Ansteuerungseinheit 3 verbunden.

Alternativ oder zusätzlich zu dem Ultraschallapplikator 1 kann auch ein optischer Laserapplikator eingesetzt werden, um die Tenderpunkte und Triggerpunkte mit Laserstrahlen anzuregen. Dabei kann das Laserlicht wahlweise mit einem niederfrequenten Signal von 20 Hz oder 50 Hz moduliert werden. Denkbar ist aber auch die Applikation eines elektrischen Niederfrequenzsignals von wahlweise 20 Hz oder 50 Hz auf die Tenderpunkte und Triggerpunkte mit einem TENS-Gerät zusätzlich zu der optischen Anregung mit Laserlicht. Die ausgewählte Zusammensetzung kann bei diesen Applikationsgeräten vor deren Verwendung auf die betroffenen Tenderpunkte und Triggerpunkte aufgetragen werden.

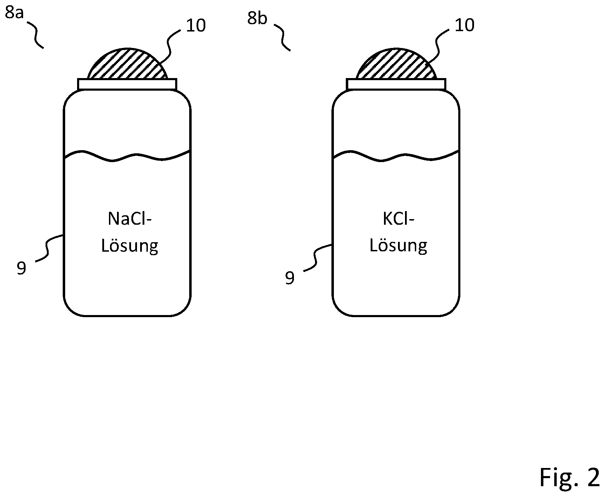

Figur 2 zeigt eine Skizze eines Sets mit zwei Applikatoren 8a, 8b in Form eines Roll-On-Stiftes.

Die Applikatoren 8a, 8b haben jeweils einen Behälter 9, in den jeweils eine wässrige Lösung eingefüllt wird. Der Behälter 9 ist jeweils mit einem Rollkopf verschlossen, der eine drehbar gelagerte Auftragerolle 10 hat. Durch Rotation der Auftragerolle 10 beim Aufdrücken auf den Bereich der Hautoberfläche, unter dem der blockierte Tenderpunkt und Triggerpunkt liegt, wird die entweder Natriumchlorid oder Kaliumchlorid als Wirkstoff enthaltende wässrige Lösung unter mechanischem Druck appliziert.

In den Behälter 9 des ersten Applikators 8a ist eine Natriumchlorid-haltige wässrige Lösung eingefüllt, um betroffene Tenderpunkte und Triggerpunkte der mit der linken Gesichtshälfte, dem rechten Arm und linken Bein zusammenhängenden Nervenbahnen, Muskel- und Faszienschlingen durch Applikation unter Druck zu behandeln.

In den Behälter 9 des zweiten Applikators 8b ist eine Kaliumchlorid-haltige wässrige Lösung eingefüllt, um betroffene Tenderpunkte und Triggerpunkte der mit der rechten Gesichtshälfte, dem linken Arm und rechten Bein zusammenhängenden Nervenbahnen, Muskel- und Faszienschlingen durch Applikation unter Druck zu behandeln.

Der Applikator 8a, 8b kann optional noch einen zusätzlichen TENS-Applikator enthalten, um beim mechanischen Auftragen der Zusammensetzung ein elektrisches Spannungssignal zu applizieren. Dieses kann auf die durch die Benetzung mit der wässrigen Lösung elektrisch leitfähige Auftragrolle 10 übertragen werden, bspw. durch eine Schleifkontaktverbindung in dem Rollkopf und ggf. durch elektrisch leitfähiges Material der Auftragrolle 10. Diese kann bspw. Graphit oder ein Metallgitter enthalten. Für den die Natriumchloridlösung enthaltenden Applikator 8a ist der TENS-Applikator zur Applikation eines niederfrequenten Signals von 50 Hz ausgebildet. Für den die Kaliumchloridlösung enthaltenden Applikator 8b ist der TENS-Applikator zur Applikation eines niederfrequenten Signals von 20 Hz ausgebildet.

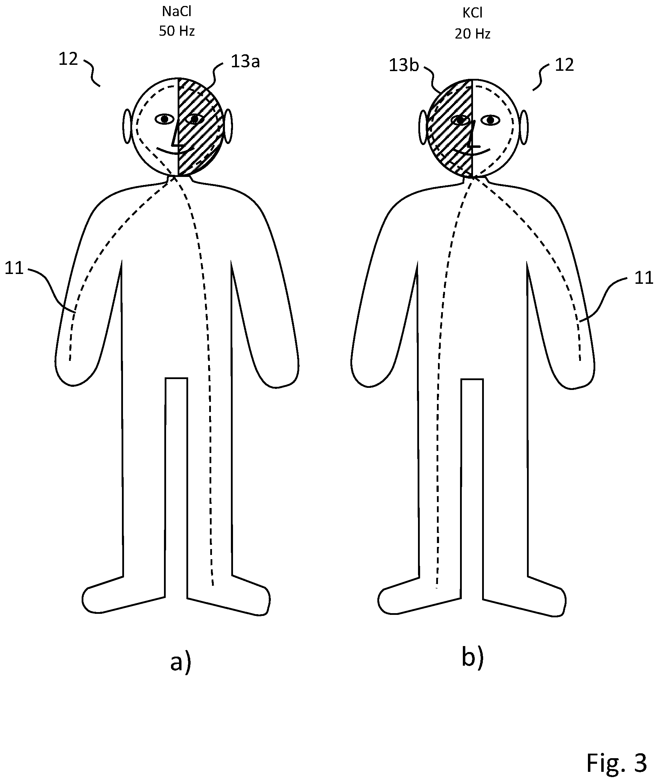

Figur 3 zeigt eine Skizze der rechten und linken Muskel- und Faszienschlingen 11 von Personen 12. Es wird deutlich, dass sich die im rechten Arm über die linke Gesichtshälfte 13a zum linken Bein verlaufenden Muskel- und Faszienschlingen 11 bei 50 Hertz auf Natrium-Chlorid reagieren. Hingegen reagieren die im linken Arm über die rechte Gesichtshälfte 13b zum rechten Bein verlaufenden Muskel- und Faszienschlingen 11 bei 20 Hertz auf Kalium-Chlorid. Dies dürfte mit der Funktion als Natrium-Kalium-Pumpe der Nervenknoten bzw. Triggerpunkte zusammenhängen.

Diese Charakteristik kann ausgenutzt werden, um die Natriumchlorid als Wirkstoff enthaltende Zusammensetzung unter Druck auf die mit der in Figur 3 a) gezeigten Muskel- und Faszienschlingen 11 anzuwenden und damit dortige Blockaden aufzulösen. Die Kaliumchlorid als Wirkstoff enthaltende Zusammensetzung wird hingegen verwendet, um diese unter Druck auf die mit der in Figur 3 b) gezeigten Muskel- und Faszienschlingen 11 anzuwenden und damit dortige Blockaden aufzulösen

In einem Ausführungsbeispiel besteht die Zusammensetzung aus den in der Tabelle 1 aufgeführten Substanzen. Zunächst werden Wollwachsalkoholsalbe und gelbes Wachs zu einer homogenen Zusammensetzung vermengt, die als Trägersubstanz dient. Die feste Substanz Natriumchlorid wird im gereinigten Wasser gelöst. Die Lösung wird der Trägersubstanz unter Rühren hinzugefügt. Schließlich wird das Brennnesselöl, das mittles einer öligen Extraktion aus Brennnesselblättern mit Hilfe von Olivenöl gewonnen wurde, ebenfalls unter Rühren hinzugefügt, sodass eine homogene Zusammensetzung entsteht. In diesem Ausführungsbeispiel ist die Zusammensetzung somit eine Salbe.

**Tabelle 1: Zusammensetzung 1**

| Substanz | Menge in 100 g Creme [g] | Massenanteil [%] |
|---|---|---|
| Natriumchlorid | 10 | 10 |
| Brennnesselöl (Olivenöl als Träger) | 28 | 28 |
| Wollwachsalkoholsalbe | 19 | 19 |
| Gelbes Wachs | 3,5 | 3,5 |
| Gereinigtes Wasser | 39,5 | 39,5 |

In einem weiteren Ausführungsbeispiel besteht die Zusammensetzung aus den in der Tabelle 2 aufgeführten Substanzen. Zunächst werden Wollwachsalkoholsalbe und gelbes Wachs zu einer homogenen Zusammensetzung vermengt, die als Trägersubstanz dient. Die feste Substanz Kaliumchlorid wird im gereinigten Wasser gelöst. Die Lösung wird der Trägersubstanz unter Rühren hinzugefügt. Schließlich wird das Brennnesselöl, das mittles einer öligen Extraktion aus Brennnesselblättern mit Hilfe von Olivenöl gewonnen wurde, ebenfalls unter Rühren hinzugefügt, sodass eine homogene Zusammensetzung entsteht. In diesem Ausführungsbeispiel ist die Zusammensetzung somit eine Salbe.

**Tabelle 2: Zusammensetzung 2**

| Substanz | Menge in 100 g Creme [g] | Massenanteil [%] |
|---|---|---|
| Kaliumchlorid | 10 | 10 |
| Brennnesselöl (Olivenöl als Träger) | 28 | 28 |
| Wollwachsalkoholsalbe | 19 | 19 |
| Gelbes Wachs | 3,5 | 3,5 |
| Gereinigtes Wasser | 39,5 | 39,5 |

**Tabelle 3: Kontroll-Zusammensetzung**

| Substanz | Menge in 100 g Creme [g] | Massenanteil [%] |
|---|---|---|
| Gereinigtes Wasser | 63,3 | 63,3 |
| Gelbes Wachs | 5,6 | 5,6 |
| Wollwachsalkoholsalbe | 31,1 | 31,1 |

Die Wirksamkeit der erfindungsgemäßen Zusammensetzungen 2 und 3 wurde in einer Kontrollreihe mit einer Kontroll-Zusammensetzung (Tabelle 3) untersucht, die eine vergleichbare Trägersubstanz wie die beispielhaften, erfindungsgemäßen Zusammensetzungen aufweist. Die Kontroll-Zusammensetzung beinhaltete dabei nicht die Wirkstoffe Natriumchlorid oder Kaliumchlorid.

### a) Tympanometrie-Messungen

Die in den Tabellen beschriebenen Zusammensetzungen mit den Wirkstoffen Natriumchlorid oder Kaliumchlorid aus beiden Ausführungsbeispielen wurden zum Wirksamkeitstest an neun (9) Patienten mit Druckausgleichsstörungen des Mittelohrs angewendet. Dabei klagten alle Patienten über ein unangenehmes Druckgefühl, eine verminderte Hörleistung und Schmerzen im betroffenen Ohr. Bei der Auswahl der Patienten wurde sichergestellt, dass keine Ausschlussgründe wie etwa mechanisch verlegte Atemwege oder Verletzungen vorlagen. In der Kontrollreihe wurde die Kontroll-Zusammensetzung an zehn (10) Patienten getestet, die nach analogen Kriterien ausgewählt wurden.

Zunächst wurde mittels Palpation untersucht, ob der Musculus tensor tympani des betroffenen Ohrs schmerzhaft kontrahiert ist. Anschließend wurden die Druckverhältnisse zwischen Umgebung und Mittelohr durch eine Tympanometrie des betroffenen Ohrs ermittelt. Eine messbare Druckausgleichsstörung lag dann vor, wenn der angelegte Druck, während der Tympanometrie, bei dem das Trommelfell die maximale Flexibilität (Compliance) zeigte, nicht im Normbereich von 0 bis -83 daPa sondern im positiven Bereich lag.

Nach Abschluss der Eingangsuntersuchung, wurde überprüft ob auf der gegenüberliegenden Körperseite eine Faszienverkürzung und Nervenbahnblockade im Bereich des Nervus facialis vorlag. Zusätzlich wurden die Bereiche des Nervus trigeminus und des Nervus medianus auf Faszienverkürzungen und Nervenbahnblockaden untersucht. Der Nachweis einer Faszienverkürzung und Nervenbahnblockade wurde in diesem Beispiel durch den Nachweis mindestens eines Tenderpunkts und Triggerpunkts im Bereich des Musculus temporalis, des Musculus occipitofrontalis, des Musculus tensor tympani oder des Musculus flexor pollicis longus erbracht.

Sofern eine Nervenbahnblockade und Faszienverkürzung auf der rechten Körperseite festgestellt wurde, wurde Salbe 1, also die Salbe mit Natriumchlorid, verwendet. Eine etwa haselnussgroße Menge der Salbe wurde auf den Ultraschallkopf eines Ultraschallgeräts aufgetragen und unter der Anwendung von Ultraschall und einer aufmodulierten Frequenz von 50 Hertz auf die gefundenen Nervenbahnblockade und den Tenderpunkte im Musculus occipitofrontalis und des Musculus flexor pollicis longus aufgetragen. Dies wurde so lange durchgeführt, bis kein Tenderpunkt oder eine Nervenbahnblockade mehr nachgewiesen werden konnte. Analog dazu wurde bei Patienten der Kontrollreihe die Kontroll-Zusammensetzung aufgetragen. Der Ultraschall wurde hier für vier (4) Minuten angewendet, da nicht von einer Auflösung des Tenderpunkts und der Nervenbahnblockade ausgegangen werden konnte.

Sofern eine Faszienverkürzung oder Nervenbahnblockade auf der linken Körperseite festgestellt wurde, wurde Salbe 2, also die Salbe mit Kaliumchlorid, verwendet. Eine etwa haselnussgroße Menge der Salbe wurde auf den Ultraschallkopf eines Ultraschallgeräts aufgetragen und unter der Anwendung von Ultraschall und einer aufmodulierten Frequenz von 20 Hertz auf die gefundenen Tenderpunkte und Nervenbahnblockaden im Musculus occipitofrontalis und des Musculus flexor pollicis longus aufgetragen. Dies wurde so lange durchgeführt, bis keine Nervenbahnblockade oder Tenderpunkte mehr nachgewiesen werden konnte. Analog dazu wurde bei Patienten der Kontrollreihe die Kontroll-Zusammensetzung aufgetragen. Der Ultraschall wurde hier für vier (4) Minuten angewendet, da nicht von einer Auflösung des Tenderpunkts und der Nervenbahnblockade ausgegangen werden konnte.

Im Anschluss pausierten die Patienten für 15 Minuten. Dabei wurde kontrolliert, dass die Patienten keine aktiven Druckausgleichsübungen, wie etwa starkes, wiederholtes Schlucken oder Gähnen, sowie Luft anhalten ausführten.

Abschließend wurde erneut eine Tympanometrie durchgeführt und der Musculus tensor tympani auf Kontraktion hin untersucht.

Die Tympanometrie-Daten nach der Verwendung der erfindungsgemäßen Zusammensetzungen mit den Wirkstoffen Natriumchlorid oder Kaliumchlorid (Tabelle 4) zeigten, dass der Druck der maximalen Trommelflexibilität nach der Verwendung der beschriebenen Salben bei 5 der 9 Patienten im Normbereich lag. Bei zwei (2) Patienten (Patient 3 und Patient 5) zeigte die Tympanometrie, dass sich der Druck der maximalen Trommelfellflexibilität vom Grenz- in den Normbereich verschoben hat. Bei zwei (2) Patienten (Patient 6 und Patient 9) konnte kein Unterschied gemessen werden (Daten nicht gezeigt). Alle neun (9) Patienten zeigten nach der Verwendung der beschriebenen Salben keinen schmerzhaft kontrahierten Musculus tensor tympani mehr. Alle neun (9) Patienten berichteten, dass die beschriebenen Beschwerden nach der Anwendung nicht mehr bestehen.

**Tabelle 4: Tympanometrie-Messung nach Anwendung erfindungsgemäßer Zusammensetzungen**

| Patient | Tympanometrie-Messung [daPa]* betroffenes Ohr | |
|---|---|---|
| | Vor Anwendung | Nach Anwendung |
| 1 | 15 | -11 |
| 2 | 110 | - 90 |
| 3 | 0 | - 16 |
| 4 - rechtes Ohr | 94 | - 41 |
| 4 - linkes Ohr | 123 | - 19 |
| 5 | 0 | - 29 |
| 7 | 141 | - 3 |
| 8 | 5 | - 16 |

| | | |
|---|---|---|
| *daPa = Dekapascal | | |

In der Kontrollreihe gemessene Tympanometrie-Daten im betroffenen Ohr (Tabelle 5) vor der Anwendung zeigen, dass der Druck der maximalen Trommelfellflexibilität bei allen Patienten an der oberen Grenze des Normbereiches (0 daPa bis -83 daPa) liegt. Das Vorhandensein einer schmerzhaften Palpation des Musculus tensor tympani und das dadurch hervorgerufene Druckgefühl im betroffenen Ohr wurde bei allen Patienten festgestellt. Nach der Anwendung der Kontroll-Zusammensetzung ohne die entsprechenden Wirkstoff Natriumchlorid oder Kaliumchlorid lag der Druck der maximalen Trommelfellflexibilität weiterhin im oberen Grenzbereich. Lediglich Patient 1 zeigte eine Verbesserung in den Normbereich hinein. Nach der Anwendung der Kontroll-Zusammensetzung wurde die schmerzhafte Palpation des Musculus tensor tympani und das Druckgefühl im Ohr bei den Patienten nicht wesentlich gelindert.

Die Tympanometrie-Daten der Kontrollreihe am gesunden Ohr (Tabelle 6) ohne jegliche Anwendung einer Zusammensetzung zeigen ebenfalls eine Schwankung der gemessenen Werte, die mit der Schwankung der Werte in Tabelle 5 vergleichbar ist. Die in der KontrollMessung am betroffenen Ohr (Tabelle 5) beobachteten Schwankungen lassen sich somit vermutlich durch die Messungenauigkeit zwischen zwei Tympanometrie-Messungen mit einer 15-minütigen Pause zwischen den Messungen erklären. Die Anwendung der Kontroll-Zusammensetzung hat somit keine vorteilhafte Wirkung auf die Werte der Tympanometrie-Messung am betroffenen Ohr. Die in der Tabelle 4 gezeigten Messwerte haben sich nach der Anwendung der erfindungsgemäßen Zusammensetzung mit den jeweiligen Wirkstoffen Natriumchlorid oder Kaliumchlorid hingegen stark verbessert. Es wurde beobachtet, dass nach der Anwendung der Druck der maximalen Trommelfellflexibilität bei dem Großteil der Patienten weit in den Normbereich hineinverschoben wurde. Außerdem berichteten alle Patienten, dass die eingangs festgestellten Beschwerden (Tender points und Druckausgleichsstörung) nach der Anwendung der erfindungsgemäßen Zusammensetzung gelindert wurden.

**Tabelle 5: Kontrollreihe - betroffenes Ohr**

| Patient | Tympanometrie-Messung in [daPa] betroffenes Ohr | |
|---|---|---|
| | vor Anwendung | nach Anwendung |
| 1 | - 27 | - 83 |
| 2 | 1 | - 12 |
| 3 | - 11 | 2 |
| 4 | - 18 | - 8 |
| 5 | - 8 | - 11 |
| 6 | - 13 | - 20 |
| 7 | - 35 | - 9 |
| 8 | - 8 | 0 |
| 9 | - 18 | - 13 |
| 10 | - 9 | - 13 |

**Tabelle 6: Kontrollreihe - gesundes Ohr**

| Patient | Tympanometrie-Messung in [daPa] gesundes Ohr | |
|---|---|---|
| | vor Anwendung* | nach Anwendung* |
| 1 | - 53 | - 11 |
| 2 | -6 | -6 |
| 3 | - 7 | - 48 |
| 4 | 8 | - 16 |
| 5 | - 27 | - 11 |
| 6 | - 7 | - 7 |
| 7 | - 21 | - 9 |
| 8 | 2 | - 2 |
| 9 | 2 | - 23 |
| 10 | - 6 | - 26 |

| | | |
|---|---|---|
| * Anwendung Kontroll-Zusammensetzung am betroffenen Ohr | | |

Die Verwendung der erfindungsgemäßen Zusammensetzungen führte zu einer starken Verbesserung der Tympanometrie-Messwerte. Außerdem konnten die Beschwerden der Patienten gelindert werden. Die Kontroll-Zusammensetzung ohne die Wirkstoffe Natriumchlorid oder Kaliumchlorid zeigte keine Verbesserung der Tympanometrie-Daten. Die beobachteten Schwankungen lassen sich mit der Messungenauigkeit zwischen zwei Messungen erklären. Dies zeigt die Wirksamkeit der erfindungsgemäßen Zusammensetzung mit den Wirkstoffen Natriumchlorid oder Kaliumchlorid zur Verwendung für die Auflösung von Nervenbahnblockaden im Beriech des Nervus trigeminus, des Nervus facialis und des Nervus medianus.

### b) Handkraft-Messungen

Eine Beschwerde, die mit dem gestörten Druckausgleich des Innenohres einhergeht, ist die Verringerung der Muskelkraft auf der gegenüberliegenden Körperhälfte der betroffenen Faszienseite. Mittels Handkraftmessung soll die Wirksamkeit der erfindungsgemäßen Zusammensetzung zur Verwendung für die Auflösung von Faszienverkürzungen gezeigt werden, indem die Zusammensetzung im Bereich des N. trigeminus, des N. facialis oder des N. medianus auf die Haut aufgetragen wird. Eine Zunahme der Handkraft zeigt die wirksame Auflösung der Faszienverkürzung in der entsprechenden Körperhälfte nach der Anwendung der erfindungsgemäßen Zusammensetzung.

In der Vergleichsstudie wurde die Handkraft von 17 Teilnehmern mit Hilfe eines "Baseline Digitaler Handkraftmesser Klinik" (MVS in Motion, Roselare, Belgien) gemessen. Zugelassen wurden nur Teilnehmer mit einer Druckausgleichsstörung im Ohr und einer Störung im Nervus trigeminus mit positiven Tender points:
i) M. temporalis (zweiter Tender point) auf der gestörten Druckausgleichsseite
ii) M. occipitofrontalis und M. flexor pollicis longus auf der Faszienseite

Die Teilnehmer befanden sich dabei in einer Sitzposition mit einer 90° Beugung im Ellenbogengelenk in Neutral-Null Stellung der Hand. Die Teilnehmer drückten mit der entsprechenden Hand für vier (4) Sekunden maximal zu. Dabei wurde der Maximalwert in Kilogramm dokumentiert. Lag die Druckausgleichsstörung auf der linken Seite vor, so wurde die Kraftmessung an der rechten Hand durchgeführt, lag die Druckausgleichsstörung auf der rechten Körperseite vor, so wurde die Kraftmessung an der linken Hand durchgeführt. Die 17 Teilnehmer wurden zufällig in zwei (2) Gruppen aufgeteilt: Gruppe 1 (9 Teilnehmer) und Gruppe 2 (8 Teilnehmer).

**Tabelle 4: Studienteilnehmer Kraftmessung**

| Patient | Gruppe | Geschlecht | Alt er | Druckausgleichsstörung Ohr | Kraftmessung Hand | Zusammensetzung |
|---|---|---|---|---|---|---|
| 1 | | weiblich | 59 | links | rechts | 1 - Na |
| 2 | | männlich | 42 | links | rechts | 1 - Na |
| 3 | | männlich | 55 | rechts | links | 2 - K |
| 4 | | männlich | 57 | rechts | links | 2 - K |
| 5 | 1 | weiblich | 35 | links | rechts | 1 - Na |
| 6 | | männlich | 24 | links | rechts | 1 - Na |
| 7 | | weiblich | 58 | links | rechts | 1 - Na |
| 8 | | weiblich | 50 | links | rechts | 1 - Na |
| 9 | | männlich | 42 | links | rechts | 1 - Na |
| 10 | 2 | weiblich | 40 | rechts | links | 2 - K* |
| 11 | | männlich | 52 | rechts | links | 2 - K* |
| 12 | | männlich | 50 | rechts | links | 2 - K* |
| 13 | | männlich | 66 | rechts | links | 2 - K* |
| 14 | | männlich | 67 | links | rechts | 1 - Na* |
| 15 | | männlich | 62 | links | rechts | 1 - Na* |
| 16 | | männlich | 60 | links | rechts | 1 - Na* |
| 17 | | weiblich | 50 | links | rechts | 1 - Na* |

| | | | | | | |
|---|---|---|---|---|---|---|
| * Zusammensetzung in Anwendung 2; Anwendung 1 mit Kontroll-Zusammensetzung | | | | | | |

In der Gruppe 1 erfolgte zunächst die oben beschriebene Kraftmessung (1. Messung). Anschließend wurde die erfindungsgemäße Zusammensetzung 1 mit dem Wirkstoff Natriumchlorid (Tabelle 1) bei Teilnehmern mit einer Druckausgleichsstörung des linken Ohres im Bereich des N. trigeminus und des N. facialis im Bereich des Tender points im M. occipitofrontalis auf der rechten Körperhälfte auf die Haut aufgetragen. Anschließend erfolgte die Anwendung von Ultraschall mit einem Puls von 50 Hz und 1 Watt Stärke für vier (4) Minuten. Bei Teilnehmern mit einer Druckausgleichsstörung des rechten Ohres wurde hingegen die erfindungsgemäße Zusammensetzung 2 mit dem Wirkstoff Kaliumchlorid (Tabelle 2) im entsprechenden Bereich auf der linken Körperhälfte auf die Haut aufgetragen. Dabei erfolgte die anschließende Anwendung von Ultraschall mit einem Puls von 20 Hz und 1 Watt Stärke für vier (4) Minuten. Nach einer 10-minütigen Pause, die der Regeneration der Muskelkraft diente, erfolgte eine erneute Kraftmessung (2. Messung).

Bei sieben (7) der neun (9) Teilnehmer zeigte sich eine erhöhte Handkraft. Bei zwei (2) Teilnehmern verringerte sich diese leicht. Eine besonders hohe Steigerung der Handkraft ist bei den Teilnehmern 1, 2, 5 und 9 zu beobachten. Die Zunahme der Handkraft nach der Anwendung der erfindungsgemäßen Zusammensetzungen mit den Wirkstoffen Natriumchlorid oder Kaliumchlorid lässt sich auch an den jeweiligen Mittelwerten der 1. und 2. Messung beobachten.

**Tabelle 5: Kraftmessung Gruppe 1**

| Teilnehmer | 1. Messung in [kg] | 2. Messung in [kg] |
|---|---|---|
| 1 | 29,8 | 33,0 |
| 2 | 45,8 | 52,3 |
| 3 | 40,8 | 38,5 |
| 4 | 39,4 | 40,2 |
| 5 | 36,2 | 40,6 |
| 6 | 67,0 | 65,3 |
| 7 | 24,6 | 26,7 |
| 8 | 31,7 | 31,9 |
| 9 | 38,5 | 45,0 |
| Mittelwert | 39,3 | 41,5 |

In der Kontroll-Gruppe 2 erfolgte ebenfalls zunächst eine Kraftmessung (1. Messung). Anschließend wurde die Kontroll-Zusammensetzung (Tabelle 3) ohne die Wirkstoffe Natriumchlorid oder Kaliumchlorid aufgetragen. Die anschließende Anwendung von Ultraschall erfolgte danach analog zur Anwendung in Gruppe 1. Nach einer 10-minütigen Pause erfolgte eine weitere Kraftmessung (2. Messung). Im Anschluss daran erfolgte die Auftragung der erfindungsgemäßen Zusammensetzung 1 mit dem Wirkstoff Natriumchlorid (Tabelle 1) bei Teilnehmern mit einer Druckausgleichsstörung des linken Ohres im Bereich N. trigeminus und des N. facialis im Bereich des Tender points im M. occipitofrontalis auf der rechten Körperhälfte auf die Haut. Anschließend erfolgte die Anwendung von Ultraschall mit einem Puls von 50 Hz und 1 Watt Stärke für vier (4) Minuten. Bei Teilnehmern mit einer Druckausgleichsstörung des rechten Ohres wurde hingegen die erfindungsgemäße Zusammensetzung 2 mit dem Wirkstoff Kaliumchlorid (Tabelle 2) im Bereich des N. trigeminus und des N. facialis im Bereich des Tender points im M. occipitofrontalis auf der linken Körperhälfte auf die Haut aufgetragen. Dabei erfolgte die anschließende Anwendung von Ultraschall mit einem Puls von 20 Hz und 1 Watt Stärke für vier (4) Minuten. Nach einer weiteren 10-minütigen Pause erfolgte eine erneute Kraftmessung (3. Messung).

**Tabelle 6: Kraftmessung Gruppe 2**

| Patient | 1. Messung in [kg] | 2. Messung in [kg] | 3. Messung in [kg] |
|---|---|---|---|
| 1 | 31,5 | 31,5 | 33,8 |
| 2 | 39,1 | 39,1 | 49,9 |
| 3 | 42,3 | 47,7 | 51,7 |
| 4 | 39,1 | 41,6 | 43,4 |
| 5 | 38,2 | 37,5 | 39,9 |
| 6 | 48,9 | 50,2 | 57,1 |
| 7 | 35,7 | 32,6 | 34,7 |
| 8 | 36,9 | 35,3 | 34,5 |
| Mittelwert | 39,0 | 39,4 | 43,1 |

Vergleicht man die Werte der 1. und 2. Messung der Handkraft der Teilnehmer der Gruppe 2, so fällt auf, dass sich die Handkraft nur leicht erhöht. Zwischen diesen beiden Messungen wurde die Kontroll-Zusammensetzung ohne die Wirkstoffe Natriumchlorid oder Kaliumchlorid aufgetragen. Betrachtet man nun die Werte der 3. Messung so ist zu erkennen, dass hier eine stark erhöhte Handkraft vorliegt. Vor der 3. Messung wurde die jeweilige erfindungsgemäße Zusammensetzung mit den Wirkstoffen Natriumchlorid oder Kaliumchlorid aufgetragen.

In beiden Gruppen der Kraftmessung konnte eine Zunahme der Handkraft nach der Verwendung der erfindungsgemäßen Zusammensetzung beobachtet werden. Die Verwendung der Kontroll-Zusammensetzung (Gruppe 2, 2. Messung) führte hingegen nur zu einer vernachlässigbaren Zunahme. Dies zeigt die Wirksamkeit der erfindungsgemäßen Zusammensetzung für die Verwendung zur Auflösung von Faszienverkürzungen, die wiederum ursächlich für den Verlust der Muskelkraft der Teilnehmer der Vergleichsstudie war.

Diese Ergebnisse zeigen die Wirksamkeit der verwendeten Zusammensetzung zur Auflösung von Faszienverkürzungen im Bereich des Nervus trigeminus, des Nervus facialis, des Nervus medianus und des Nervus ischiadicus.

In einem weiteren Ausführungsbeispiel wird die Salbe aus den obigen Beispielen in einen Roll-On Stick, ähnlich eines Deo-Rollers, gefüllt. Da hier kein Ultraschall angewendet werden kann, hat der Roll-On Stick einen integrierten Massagekopf, um die Salbe mit Druck auf die Haut aufzutragen. Der Roll-On Stick kann unterwegs oder daheim verwendet werden, wenn sich die typischen Beschwerden der Faszienverkürzung spontan bemerkbar machen.

In einem weiteren Ausführungsbeispiel wird ein Roll-On Stick mit Salbe 1 und ein Roll-On Stick mit Salbe 2 zusammen in einem Set angeboten. So kann unterwegs oder daheim ganzheitlich auf Faszienverkürzungen in beiden Körperhälften reagiert werden.

### Bezugszeichenliste:

- 1: - Ultraschall-Applikator
- 2: - Behälter mit cremeartiger Zusammensetzung
- 2a: - Behälter mit Zusammensetzung mit NaCl als Wirkstoff
- 2b: - Behälter mit Zusammensetzung mit KCl als Wirkstoff
- 3: - Ansteuerungseinheit
- US: - Ultraschallfrequenzgenerator
- 4a, 4b: - Niederfrequenzgenerator
- 5: - Mischer
- 6: - Ultraschallaktuator
- 7: - Handgehäuse
- 8a, 8b: - Applikator
- 9: - Behälter
- 10: - Auftragerolle
- 11: - Muskel- und Faszienschlingen
- 12: - Person
- 13a, 13b: - Gesichtshälfte

## Patentansprüche

1. Zusammensetzung, die einen Träger und entweder Natriumchlorid oder Kalium-chlorid enthält, zur Verwendung für die Auflösung von Faszienverkürzungen, indem die Zusammensetzung im Bereich des Nervus trigeminus, des Nervus facialis, des Nervus medianus, des Nervus ischiadicus oder auf eine Kombination aus mindestens zwei dieser Bereiche auf die Haut aufgetragen wird.

2. Zusammensetzung nach einem der vorhergehenden Ansprüche, **dadurch gekennzeichnet, dass** die Zusammensetzung Acetylcholin und/oder Phosphat enthält.

3. Zusammensetzung nach einem der vorhergehenden Ansprüche, **dadurch gekennzeichnet, dass** der Träger mindestens eine dermatologisch annehmbare Trägersubstanz enthält.

4. Zusammensetzung nach einem der vorhergehenden Ansprüche, **dadurch gekennzeichnet, dass** die Zusammensetzung einen Extrakt aus Brennnessel beinhaltet, der Acetylcholin und Phosphat enthält.

5. Zusammensetzung nach Anspruch 4, **dadurch gekennzeichnet, dass** das Gewichtsverhältnis von entweder Natriumchlorid oder Kaliumchlorid zu Brennnesselextrakt und zu mindestens einer dermatologisch annehmbaren Trägersubstanz in einem Bereich von 1 bis 10 Gewichtsprozenten Natriumchlorid oder Kaliumchlorid, 1 bis 28 Gewichtsprozenten Brennnesselextrakt und 1 bis 62 Gewichtsprozenten mindestens einer dermatologisch annehmbaren Trägersubstanz liegt.

6. Zusammensetzung nach einem der vorhergehenden Ansprüche, **dadurch gekennzeichnet, dass** die Zusammensetzung entweder Natriumchlorid oder Kalium-chlorid mit einem Anteil von jeweils 10 Gewichtsprozent, Brennnesselextrakt mit einem Anteil von 28 Gewichtsprozent, gelbes Wachs mit einem Anteil von 3 bis 4 Gewichtsprozent, Wollwachsalkoholsalbe DAB mit einem Anteil von 18 bis 20 Gewichtsprozent, sowie gereinigtes Wasser mit einem Anteil von 38 bis 42 Gewichtsprozent umfasst.

7. Zusammensetzung nach einem der vorhergehenden Ansprüche, **dadurch gekennzeichnet, dass** die mindestens eine dermatologisch annehmbare Trägersubstanz gereinigtes Wasser ist oder ausgewählt ist aus der Gruppe der:
a. hydrophoben Salben, einschließlich der Carbogele, der Lipogele und der Silikongele;
b. wasseraufnehmenden Salben, einschließlich der Absorptionsbasen Wasser in Öl und der Absorptionsbasen Öl in Wasser;
c. hydrophilen Salben, einschließlich der festen und flüssigen Macrogole;
d. hydrophile Cremes;
e. hydrophoben Cremes;
f. hydrophoben Gele;
g. hydrophile Gele;
h. hydrophobe Pasten;
i. hydrophile Pasten.

8. Verwendung einer Zusammensetzung nach einem der Ansprüche 1 bis 7 für die Auflösung von Faszienverkürzungen durch Auftragen mit einem Applikator, der eine Druck-Auftragungseinheit zum Auftragen der Zusammensetzung unter mechanischem Druck auf die Haut hat, **dadurch gekennzeichnet, dass** die Druck-Auftragungseinheit zum Auftragen der Zusammensetzung auf die Haut ein Ultraschallapplikator mit einem zu Ultraschallschwingungen anregbarem Ultraschallkopf ist, wobei der Ultraschallapplikator zur Aufmodulation einer Frequenz von wahlweise 20 Hertz oder 50 Hertz ausgebildet ist, und dass die Zusammensetzung nach einem der Ansprüche 1 bis 7 auf den Ultraschallkopf aufgetragen wird, um wahlweise die Natriumchlorid enthaltende Zusammensetzung mit Ultraschall und einer aufmodulierten Frequenz von 50 Hertz oder die Kaliumchlorid enthaltende Zusammensetzung mit Ultraschall und einer aufmodulierten Frequenz von 20 Hertz zu applizieren.

9. Verwendung einer Zusammensetzung nach einem der Ansprüche 1 bis 7 für die Auflösung von Faszienverkürzungen durch Auftragen mit einem Applikator, **dadurch gekennzeichnet, dass** entweder eine im Wesentlichen Natriumchlorid enthaltenden Natriumchloridlösung oder eine im Wesentlichen Kaliumchlorid enthaltende Kaliumchloridlösung als Zusammensetzung nach Anspruch 1 in dem Applikator enthalten ist, wobei der Applikator eine Druck-Auftragungseinheit zum Auftragen der Zusammensetzung unter mechanischem Druck auf die Haut aufweist.

10. Verwendung nach Anspruch 9, **dadurch gekennzeichnet, dass** der Applikator ein Roll-On-Applikator mit entweder Natriumchlorid oder Kaliumchlorid und mindestens einer dermatologisch annehmbaren Trägersubstanz enthaltende Zusammensetzung nach Anspruch 1 in einem Behälter und einem eine Öffnung des Behälters abschließenden Rollkopf mit einer drehbar gelagerten Auftragrolle ist.

11. Verwendung einer Zusammensetzung nach einem der Ansprüche 1 bis 7 für die Auflösung von Faszienverkürzungen durch Auftragen mit einem Set aus einem ersten Applikator, der die Natriumchlorid enthaltende Zusammensetzung beinhaltet und zum Auftragen der Natriumchlorid enthaltenden Zusammensetzung auf die Haut ausgebildet ist und einem zweiten Applikator, der die Kaliumchlorid enthaltende Zusammensetzung beinhaltet und zum Auftragen der Kaliumchlorid enthaltenden Zusammensetzung auf die Haut ausgebildet ist, indem die Zusammensetzung zur Auflösung von Faszienverkürzungen im Bereich des Nervus trigeminus, des Nervus facialis, des Nervus medianus, des Nervus ischiadicus oder auf eine Kombination aus mindestens zwei dieser Bereiche auf die Haut aufgetragen wird.
